# EUROPEAN PATENT SPECIFICATION

(11) **EP 1 966 546 B1**
(45) Date of publication and mention of the grant of the patent: **03.12.2014**
(21) Application number: 05855722.4
(22) Date of filing: 28.12.2005
(51) Int. Cl.: F24H 1/06, A61F 7/00, F23D 14/18, A61F 7/03, F24H 1/00, F23D 14/14, F23D 99/00

(54) **PORTABLE FLUID WARMING SYSTEM**
TRAGBARES FLUIDERWÄRMUNGSSYSTEM
SYSTEME PORTATIF DE CHAUFFAGE DE FLUIDE

(43) Date of publication of application: 10.09.2008
(73) Proprietor: The Board of Regents of The University of Texas System, Austin, TX 78701 (US)
(72) Inventor: GILL, Brijesh, Houston, Texas 77008 (US); COX, Charles, Bellaire, Texas 77401 (US)
(74) Representative: Manley, Nicholas Michael
(86) International application number: PCT/US2005/047209
(87) International publication number: WO 2007/075169

(56) References cited:
- WO-A2-97/38265
- DE-A1- 19 645 143
- US-A- 1 616 143
- US-A- 2 904 014
- US-A- 4 480 631
- US-A- 4 726 767
- US-A- 5 101 804
- US-A- 5 544 645

## Description

### BACKGROUND OF THE INVENTION

### 1. Field Of The Invention

The present invention relates to a portable apparatus for warming biocompatible fluids for use in the treatment of injured patients. The present invention may be used to warm intravenous fluids for trauma resuscitation or to warm air from a ventilator circuit. The portable nature of the present invention makes it highly suitable for field applications, such as a forward surgical hospital near a combat zone.

### 2. Description Of The Prior Art

Hypothermia is quite common in injured patients, including patients experiencing trauma. Hypothermia produces a number of physiologic derangements which worsen the effects of major injury. Several relevant enzyme systems begin to lose efficiency as their ambient temperature falls. For example, the myocardium, which is dependent on the function of membrane-channel type enzymes for normal electrical function, shows a predictable series of atrial followed by ventricular arrhythmias as core temperature falls below 34°C. Cardiac output is further compromised by poor function of intrinsic myocardial components, with bovine myocardium showing a linear decrease in developed tension with decreasing temperature.

Hypothermia also exacerbates hemorrhagic shock in multiple ways. The onset of coagulopathy which accompanies hypothermia, has been shown to result from malfunction of both clotting factors and platelets.

While profound hypothermia may be tolerated by immersion or cardiac surgery patients, the presence of hypothermia in trauma patients predicts significantly higher mortality. Mortality doubles for heterogeneous groups of trauma patients at 34°C, and survival after trauma is very rare when the core temperature falls below 32°C. This effect is greater for more severely injured patients.

The development of hypothermia comes from several factors. Body heat is convectively lost to the environment, and this effect is enhanced by bleeding or the presence of large surface area bums. The body loses both central thermoregulation and peripheral shivering after traumatic injury. Less heat is produced peripherally as perfusion decreases in shock.

The administration of intravenous fluids is used in trauma resuscitation. The administration of fluid at ambient temperature, however, induces hypothermia. This condition is worse in more severely injured patients, who require the most fluid and have the least ability to tolerate the additional insult of decreased core temperature. Hypothermia and mortality clearly increase after the administration of five liters of crystalloid or five units of packed red blood cells, and the onset of hypothermia increases the incidence of coagulopathy in injured patients, particularly in the presence of acidosis.

As used herein, the term "biocompatible fluid" refers to any fluid that is appropriate for infusion into the human body, including normal saline and its less concentrated derivatives, Ringer's lactate, and hypertonic crystalloid solutions; blood and fractions of blood including plasma, platelets, albumin and cryoprecipitate; intravascular volume expanding blood substitutes including hetastarch, polymerized hemoglobin, perfluorocarbons; medications reconstituted with saline or sterile water; and medical gasses including air, oxygen, helium, nitric oxide, and combinations thereof.

Prior art methods of treating hypothermia include direct intravenous fluid warming. The fluid that is warmed may be the blood other biocompatible liquid.

Prior art devices used to warm one or more biocompatible fluids for use in the treatment of trauma have used electricity as their heating source. These systems are referred to herein as "biocompatible liquid infusion systems." Electrically heated biocompatible fluid infusion systems have several drawbacks. If the source of electrical energy is alternating current from a central generating station, the unit can then only be used in locations where such alternating current is available. This significantly limits the locations where the units may be used. Locations such as non-industrialized nations or battlefield locations are likely not have readily available sources of alternating current to power such systems.

Batteries may also be used to generate electrical energy. It is believed that sufficient power to heat a single liter of fluid to 20°C within a ten minute time period would require a rechargeable battery the size and weight of a large laptop computer. In such a case, the weight of the battery would exceed the weight of a liter of saline fluid. The size and weight of such a unit would limit its portability. Additionally, the battery would require recharging after each liter of biocompatible fluid is delivered.

German patent application DE 196 45 143 discloses a catalytic heat generator with a cylindrical burner connected to an inlet for a gas-air mixture. The exhaust fumes from the burner flow over the heat exchanger concentrically enclosing the burner chamber. The gas and air mixture inlet contains a catalytically coated flow element in the form of a reaction pipe with catalytically coated exterior. The reaction pipe is enclosed by an annular duct of the gas-air mixture inlet. the heat exchanger is in the form of a double casing through which the heat exchanger medium flows. The outer casing has plates protruding outwardly into the exhaust chamber.

US patent 4,726,767 discloses a hot blow generator utilizing liquefied gas as a source of energy. It comprises a fuel gas jet, an air/fuel gas mixer using the ejector effect of the jetting fuel gas, and a catalytic combustor. A fan blows an air stream to the air/fuel gas mixture, depending on the combustion temperature in the device.

US patent 5,101,804 discloses a portable self-contained device for warming physiological fluids. It is comprised of a reaction container which is capable of storing at least one chemical which, when mixed with a second chemical, will produce an exothermic chemical reaction, a heat exchanger to absorb heat and transfer it to the physiological fluid or blood, and temperature regulating means.

The present invention overcomes the limitations of prior art biocompatible fluid infusion systems by providing a biocompatible liquid infusion system that is not dependent upon electrical energy as a heat source. The present invention is light enough and compact enough to be used in field hospital environments which are remotely located from large central hospitals and from sources of alternating current. The present invention may also be used to warm air delivered to a patent via a ventilation circuit.

### SUMMARY OF THE INVENTION

The present invention is directed toward a portable biocompatible fluid warming system that may be used for infusing biocompatible liquids into a patient for the treatment of trauma. The present invention uses heat from hydrocarbon combustion. Hydrocarbon combustion can take place in the absence of an open flame. As an example, in one embodiment, the present invention may be used with a gaseous hydrocarbon such as butane which is allowed to flow onto a platinum mesh and then ignited. The butane combines with oxygen and liberates heat which then heats the platinum mesh. In this embodiment, the temperature of the mesh stabilizes at the ignition temperature of the butane, thereby allowing combustion to occur on the surface of the platinum mesh.

The present invention functions as a heat exchanger which takes the heat resulting from the hydrocarbon combustion process described above and transfers this heat to a biocompatible liquid.

### DESCRIPTION OF THE FIGURES

Figure 1 is a side cutaway view of one embodiment of the outer housing of the present invention.
Figure 2 is an isometric view of one embodiment of the present invention.
Figure 3 is an exploded isometric view of one embodiment of the present invention.
Figure 4 is a block diagram of the process control instrumentation of a preferred embodiment of the present invention.
Figure 5 is a side view of the gas delivery and ignition components of the present invention.
Figure 6 is a side view of one embodiment of the actuator of the present invention.

### DESCRIPTION OF THE PREFERRED EMBODIMENTS

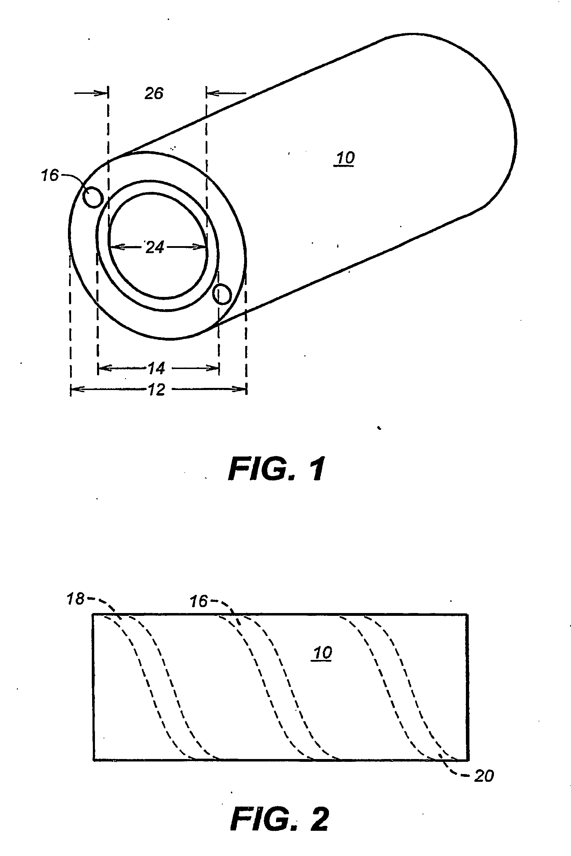

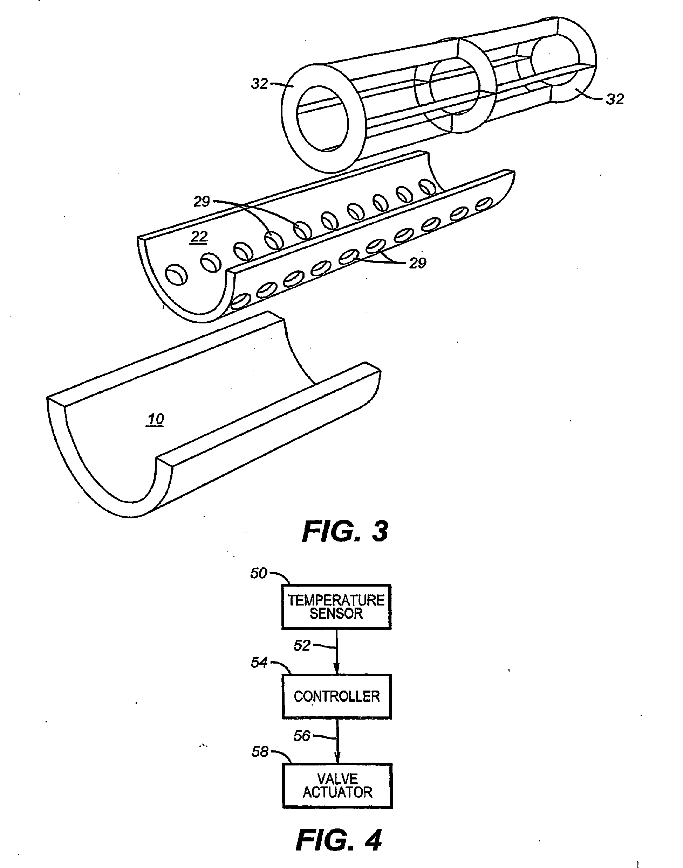

In a preferred embodiment, the present invention is directed toward a portable warmer of a biocompatible fluid comprising an outer steel housing 10 comprising a first outer diameter 12, a first inner diameter 14, and at least one flow channel 16 located between the first inner diameter and the first outer diameter as shown in Figs. 2 and 3.

The term "diameter" as used herein refers to the length of an axis which bisects a cross sectional area of the housing. For cylindrical geometries the diameter is constant at a given point along the longitudinal axis of the cylindrical housing at various azimuths. For noncylindrical geometries the diameter at a given point along the longitudinal axis of the housing may vary as a function of the azimuth.

In a preferred embodiment, the outer diameter of the steel housing is no more than 20 centimeters. In another preferred embodiment, the outer housing is cylindrical. In another preferred embodiment, the outer housing is made of stainless steel.

The flow channel comprises an inlet section 18 and an outlet section 20, as shown in Fig. 1. In a preferred embodiment, the flow channel is helical, as shown in Fig. 1. In another preferred embodiment, the mass of the portable warmer described herein is less than or equal to two kilograms.

This embodiment of the invention further comprises an inner aluminum housing 22 having a second outer diameter 24 sized to fit snugly within said outer housing and an inner wall defining a second inner diameter 26 and an internal volume as shown in Figs. 2 and 3. In the preferred embodiment depicted in Fig. 2, the internal volume defined by inner diameter 26 extends longitudinally the length of outer housing 10. In a preferred embodiment both the outer and inner housings are cylindrical. In a preferred embodiment, the inner housing comprises at least two ports 29 to permit fluid flow between regions on opposite sides of the inner housing as shown in Fig. 3. In another preferred embodiment, the inner housing comprises at least two grooves in which fluid can flow.

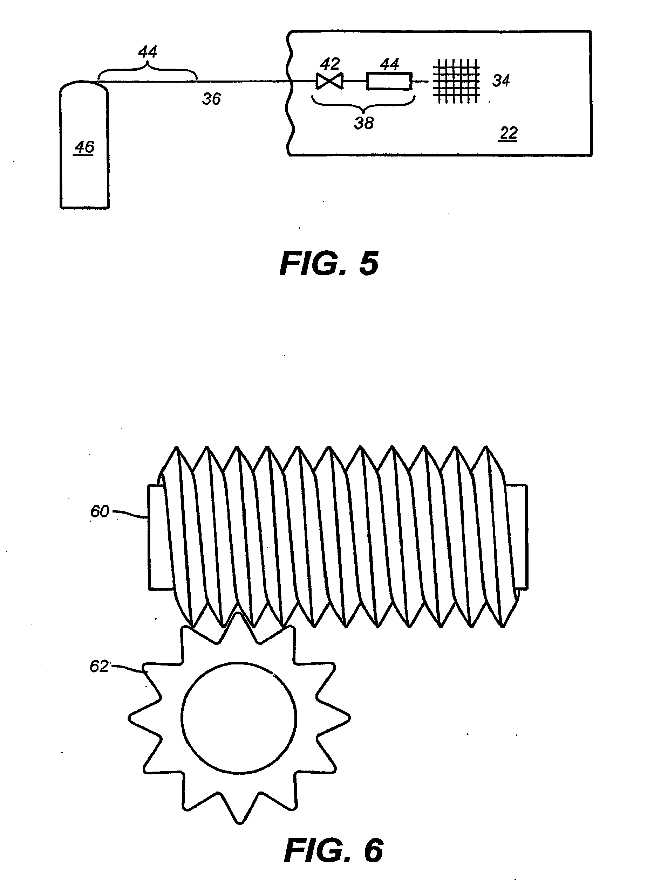

This preferred embodiment further comprises a multiplicity of heat transfer protrusions 32 affixed to the inner wall as shown in Fig. 3. In one preferred embodiment, the heat transfer protrusions are fins. In another preferred embodiment, the heat transfer protrusions are ring like disks as shown in Fig. 3. In a preferred embodiment, the invention further comprises a metallic mesh 34 located within the internal cylindrical volume as shown in Fig. 5. In a preferred embodiment, the metallic mesh is made from a metal selected from the group consisting of palladium and platinum.

This invention further comprises a gas delivery line 36 comprising a distal end region 38 located within the internal volume and a proximal end region 40 located outside the internal volume as shown in Fig. 5. A valve 42 is located in the gas delivery line. In a preferred embodiment, the valve is a needle valve.

The invention further comprises a spark igniter 44 located in the internal volume and situated close enough to the valve such that when the valve is open and gas flows through the gas delivery line and the valve into the internal volume, the igniter can ignite the gas and cause the wire mesh to be heated to a temperature in excess of 420°C, as shown in Fig. 5. In a preferred embodiment, the invention further comprises a source of combustible gaseous hydrocarbon 46 in fluid communication with the proximal end of the gas delivery line as shown in Fig. 5. In a preferred embodiment, the gaseous hydrocarbon is selected from the group consisting of methane, ethane, propane, and butane.

Another embodiment of the present invention comprises process controls for controlling the temperature of the fluid output from the portable fluid warmer. In this embodiment, the invention further comprises a temperature sensor 50 positioned to sense the temperature of a fluid flowing through the outlet section of the flow channel and to transmit a temperature signal 52 indicative of the temperature of a fluid flowing through the outlet section of the flow channel as shown in Fig. 4. In a preferred embodiment, the temperature sensor is selected from the group consisting of a thermistor, a thermocouple, and a solid state thermal sensor.

In another preferred embodiment, the invention further comprises a controller 54 operatively connected to receive the temperature signal from the sensor and transmit a control signal 56 responsive to the temperature signal as shown in Fig. 4. In one preferred embodiment, the controller is a microcontroller. In another preferred embodiment, the controller is an analog controller. In a preferred embodiment, when the temperature signal indicates that the temperature of the fluid flowing through the outlet section of flow channel exceeds a preselected temperature threshold, a control signal to increase the degree of closure of the valve is generated. In another preferred embodiment, the actuator comprises worm gear 60 mechanically coupled to a spur gear 62 as shown in Fig. 6. In this embodiment, the spur gear is mechanically coupled to the valve.

In this embodiment, the invention further comprises a valve actuator 58 operatively connected to the valve and to the controller to control the degree of closure of the valve in response to the control signal as shown in Fig. 4. In a preferred embodiment, the valve actuator is coupled to receive the control signal from the controller. In a preferred embodiment, the valve actuator is a servo-controller.

In other embodiments, temperature may be regulated by controlling fuel flow into the inner cylinder. Additionally, temperature may be controlled by mixing small amounts of unheated fluid with the heated fluid exiting the portable warming device. In another embodiment, fluid temperature may be controlled by changing the thermal conductance of the layer between the inner cylinder and the flow channels.

The foregoing disclosure and description of the invention are illustrative and explanatory. Various changes in the size, shape, and materials, as well as in the details of the illustrative construction may be made without departing from the scope of the invention as defined in the claims.

## Claims

1. A portable warmer of a biocompatible fluid comprising:
(a) an outer stainless steel housing having a first outer diameter, a first inner diameter, and at least one flow channel for the biocompatible fluid located between said first inner diameter and said first outer diameter, said flow channel comprising an inlet section and an outlet section;
(b) an inner aluminum housing having a second outer diameter sized to fit snugly within said outer housing and an inner wall defining a second inner diameter and an internal cylindrical volume;
(c) a multiplicity of heat transfer protrusions affixed to said inner wall;
(d) a metallic mesh located within said internal volume;
(e) a gas delivery line comprising a distal end region located within said internal volume and a proximal end region located outside said internal volume;
(f) a valve located in the distal end region of said gas delivery line; and
(g) a spark igniter located in said internal volume and situated close enough to said valve such that when said valve is open and gas flows through said gas delivery line and said valve into said internal volume, the igniter can ignite the gas and cause the gas to combust, thereby heating the metallic mesh to a temperature in excess of 420 degrees Centigrade.

2. The device of claim 1, wherein the metallic mesh is made from a metal selected from the group consisting of palladium and platinum.

3. The device of claim 1, wherein said flow channel is helical.

4. The device of claim 1, wherein the mass of the portable warmer is less than or equal to 2 kilograms.

5. The device of claim 1, wherein the outer diameter of the steel housing is no more than 20 centimeters.

6. The device of claim 1, wherein said heat transfer protrusions are disc like rings.

7. The device of claim 1, further comprising a source of combustible gaseous hydrocarbon in fluid communication with said proximal end.

8. The device of claim 7, wherein said gaseous hydrocarbon is selected from the group consisting of methane, ethane, propane, and butane.

9. The device of claim 1, further comprising:
(a) a temperature sensor positioned to sense the temperature of a fluid flowing through the outlet section of said flow channel and to transmit a temperature signal indicative of the temperature of a fluid flowing through the outlet section of said flow channel;
(b) a controller operatively connected to receive said temperature signal from said sensor and to transmit a control signal responsive to said temperature signal; and
(c) a valve actuator operatively connected to said valve and to said controller to control the degree of closure of said valve in response to said control signal.

10. The device of claim 9, wherein said temperature sensor is selected from the group consisting of a thermistor, a thermocouple and a solid state thermal sensor.

11. The device of claim 9, wherein a control signal to increase the degree of closure of said valve is generated when the temperature signal indicates that the temperature of said fluid flowing through the outlet section of said flow channel exceeds a preselected temperature threshold.

12. The device of claim 11, wherein the controller is a microcontroller and the valve actuator is a servo-controller.

13. The device of claim 9, wherein the controller is an analog controller.

14. The device of claim 9, wherein said actuator comprises a worm gear mechanically coupled to a spur gear.

15. The device of claim 1, wherein said inner housing comprises at least two ports to permit fluid flow between regions on opposite sides of said inner cylinder.

16. The device of claim 1, wherein said inner housing comprises at least two grooves in which fluid can flow.

17. The device of claim 1, wherein said outer housing is cylindrical.

18. The device of claim 17, wherein said inner housing is cylindrical.

19. The device of claim 18, further comprising:
(a) a temperature sensor positioned to sense the temperature of a fluid flowing through the outlet section of said flow channel and to transmit a temperature signal indicative of the temperature of a fluid flowing through the outlet section of said flow channel;
(b) a controller operatively connected to receive said temperature signal from said sensor and to transmit a control signal responsive to said temperature signal; and
(c) a valve actuator operatively connected to said valve and to said controller to control the degree of closure of said valve in response to said control signal.

## Patentansprüche

1. Tragbare Erwärmungsvorrichtung eines biokompatiblen Fluids, die Folgendes umfasst:
(a) ein äußeres Edelstahlgehäuse mit einem ersten Außendurchmesser, einem ersten Innendurchmesser und mindestens einem Flussdurchgang für das biokompatible Fluid, der sich zwischen dem ersten Innendurchmesser und dem ersten Außendurchmesser befindet, wobei der Flussdurchgang einen Einlassabschnitt und einen Auslassabschnitt umfasst;
(b) ein inneres Aluminiumgehäuse mit einem zweiten Außendurchmesser, der dazu bemessen ist, festsitzend in das äußere Gehäuse zu passen und einer Innenwand, die einen zweiten Innendurchmesser und ein innenliegendes zylindrisches Volumen definiert;
(c) eine Vielzahl von Wärmeübertragungsvorsprüngen, die an der Innenwand befestigt sind;
(d) ein Metallgitter, das sich innerhalb von dem innenliegenden Volumen befindet;
(e) eine Gaszufuhrleitung, die einen innerhalb von dem innenliegenden Volumen befindlichen distalen Endbereich und einen außerhalb von dem innenliegenden Volumen befindlichen proximalen Endbereich umfasst;
(f) ein Ventil, das sich in dem distalen Endbereich der Gaszufuhrleitung befindet; und
(g) eine Funkenzündvorrichtung, die sich in dem innenliegenden Volumen befindet und nah genug bei dem Ventil situiert ist, dass, wenn das Ventil offen ist und Gas durch die Gaszufuhrleitung und das Ventil in das innenliegende Volumen fließt, die Zündvorrichtung das Gas entzünden kann und das Gas veranlassen kann, zu verbrennen, wodurch das Metallgitter auf eine Temperatur von mehr als 420 Grad Celsius erwärmt wird.

2. Vorrichtung nach Anspruch 1, wobei das Metallgitter aus einem Metall hergestellt ist, das aus der aus Palladium und Platin bestehenden Gruppe ausgewählt wird.

3. Vorrichtung nach Anspruch 1, wobei der Flussdurchgang schneckenförmig ist.

4. Vorrichtung nach Anspruch 1, wobei die Masse der tragbaren Erwärmungsvorrichtung kleiner oder gleich 2 Kilogramm ist.

5. Vorrichtung nach Anspruch 1, wobei der Außendurchmesser des Stahlgehäuses nicht mehr als 20 Zentimeter beträgt.

6. Vorrichtung nach Anspruch 1, wobei es sich bei den Wärmeübertragungsvorsprüngen um scheibenartige Ringe handelt.

7. Vorrichtung nach Anspruch 1, weiter umfassend eine Quelle von verbrennbarem gasförmigem Kohlenwasserstoff in Fluidverbindung mit dem proximalen Ende.

8. Vorrichtung nach Anspruch 7, wobei der gasförmige Kohlenwasserstoff aus der aus Methan, Ethan, Propan und Butan bestehenden Gruppe ausgewählt wird.

9. Vorrichtung nach Anspruch 1, die weiter Folgendes umfasst:
(a) einen Temperatursensor, der dazu positioniert ist, die Temperatur eines durch den Auslassabschnitt des Flussdurchgangs fließenden Fluids abzufühlen und ein für die Temperatur eines durch den Auslassabschnitt des Flussdurchgangs fließenden Fluids indikatives Temperatursignal zu senden;
(b) einen Kontroller, der wirksam angeschlossen ist, um das Temperatursignal von dem Sensor zu empfangen und ein auf das Temperatursignal ansprechendes Steuersignal zu senden; und
(c) einen Ventilantrieb, der wirksam mit dem Ventil und mit dem Kontroller verbunden ist, um den Grad des Schließens des Ventils als Reaktion auf das Steuersignal zu steuern.

10. Vorrichtung nach Anspruch 9, wobei der Temperatursensor aus der aus einem Thermistor, einem Thermopaar und einem Festkörper-Thermosensor bestehenden Gruppe ausgewählt wird.

11. Vorrichtung nach Anspruch 9, wobei ein Steuersignal zum Erhöhen des Grads des Schließens des Ventils erzeugt wird, wenn das Temperatursignal angibt, dass die Temperatur des durch den Auslassabschnitt des Flusskanals fließenden Fluids einen vorausgewählten Temperaturschwellenwert übersteigt.

12. Vorrichtung nach Anspruch 11, wobei es sich bei dem Kontroller um einen Mikrokontroller handelt und es sich bei dem Ventilantrieb um einen Servokontroller handelt.

13. Vorrichtung nach Anspruch 9, wobei es sich bei dem Kontroller um einen Analogkontroller handelt.

14. Vorrichtung nach Anspruch 9, wobei der Antrieb ein mechanisch an ein Stirnrad gekoppeltes Schneckenrad umfasst.

15. Vorrichtung nach Anspruch 1, wobei das innere Gehäuse mindestens zwei Öffnungen umfasst, um den Fluidfluss zwischen Bereichen auf gegenüberliegenden Seiten des inneren Zylinders zuzulassen.

16. Vorrichtung nach Anspruch 1, wobei das innere Gehäuse mindestens zwei Rillen umfasst, in denen Fluid fließen kann.

17. Vorrichtung nach Anspruch 1, wobei das äußere Gehäuse zylindrisch ist.

18. Vorrichtung nach Anspruch 17, wobei das innere Gehäuse zylindrisch ist.

19. Vorrichtung nach Anspruch 18, die weiter Folgendes umfasst:
(a) einen Temperatursensor, der dazu positioniert ist, die Temperatur eines durch den Auslassabschnitt des Flussdurchgangs fließenden Fluids abzufühlen und ein für die Temperatur eines durch den Auslassabschnitt des Flussdurchgangs fließenden Fluids indikatives Temperatursignal zu senden;
(b) einen Kontroller, der wirksam angeschlossen ist, um das Temperatursignal von dem Sensor zu empfangen und ein auf das Temperatursignal ansprechendes Steuerungssignal zu senden; und
(c) einen Ventilantrieb, der wirksam mit dem Ventil und mit dem Kontroller verbunden ist, um den Grad des Schließens des Ventils als Reaktion auf das Steuersignal zu steuern.

## Revendications

1. Dispositif de chauffage portable pour un fluide biocompatible comportant :
(a) un logement extérieur en acier inoxydable ayant un premier diamètre extérieur, un premier diamètre intérieur, et au moins un canal d'écoulement pour le fluide biocompatible situé entre ledit premier diamètre intérieur et ledit premier diamètre extérieur, ledit canal d'écoulement comportant une section d'entrée et une section de sortie ;
(b) un logement intérieur en aluminium ayant un deuxième diamètre extérieur dimensionné à des fins d'ajustement parfait à l'intérieur dudit logement extérieur et une paroi intérieure définissant un deuxième diamètre intérieur et un volume cylindrique interne ;
(c) une multiplicité de parties saillantes de transfert de chaleur fixées sur ladite paroi intérieure ;
(d) un grillage métallique situé à l'intérieur dudit volume interne ;
(e) une conduite de distribution de gaz comportant une région d'extrémité distale située à l'intérieur dudit volume interne et une région d'extrémité proximale située à l'extérieur dudit volume interne ;
(f) une soupape située dans la région d'extrémité distale de ladite conduite de distribution de gaz ; et
(g) un allumeur à étincelles situé dans ledit volume interne et situé suffisamment près de ladite soupape de sorte que, quand ladite soupape est ouverte et que le gaz s'écoule au travers de ladite conduite de distribution de gaz et de ladite soupape dans ledit volume interne, l'allumeur peut allumer le gaz et entraîner la combustion du gaz, pour ainsi chauffer le grillage métallique jusqu'à une température dépassant 420 degrés Centigrade.

2. Dispositif selon la revendication 1, dans lequel le grillage métallique est réalisé en un métal sélectionné dans le groupe constitué par du palladium et du platine.

3. Dispositif selon la revendication 1, dans lequel ledit canal d'écoulement est hélicoïdal.

4. Dispositif selon la revendication 1, dans lequel la masse du dispositif de chauffage portable est inférieure ou égale à 2 kilogrammes.

5. Dispositif selon la revendication 1, dans lequel le diamètre extérieur du logement en acier ne mesure pas plus de 20 centimètres.

6. Dispositif selon la revendication 1, dans lequel lesdites parties saillantes de transfert de chaleur sont des anneaux similaires à des disques.

7. Dispositif selon la revendication 1, comportant par ailleurs une source d'hydrocarbure gazeux combustible en communication fluidique avec ladite extrémité proximale.

8. Dispositif selon la revendication 7, dans lequel ledit hydrocarbure gazeux est sélectionné dans le groupe constitué par du méthane, de l'éthane, du propane, et du butane.

9. Dispositif selon la revendication 1, comportant par ailleurs :
(a) un capteur de température positionné pour capter la température d'un fluide s'écoulant au travers de la section de sortie dudit canal d'écoulement et pour transmettre un signal de température indiquant la température d'un fluide s'écoulant au travers de la section de sortie dudit canal d'écoulement ;
(b) un dispositif de commande connecté fonctionnellement pour recevoir ledit signal de température en provenance dudit capteur et pour transmettre un signal de commande en réponse audit signal de température ; et
(c) un actionneur de soupape connecté fonctionnellement à ladite soupape et audit dispositif de commande pour commander le degré de fermeture de ladite soupape en réponse audit signal de commande.

10. Dispositif selon la revendication 9, dans lequel ledit capteur de température est sélectionné dans le groupe constitué par une thermistance, un thermocouple et un capteur thermique à semi-conducteurs.

11. Dispositif selon la revendication 9, dans lequel un signal de commande destiné à augmenter le degré de fermeture de ladite soupape est généré quand le signal de température indique que la température dudit fluide s'écoulant au travers de la section de sortie dudit canal d'écoulement dépasse un seuil de température présélectionné.

12. Dispositif selon la revendication 11, dans lequel le dispositif de commande est un microcontrôleur et l'actionneur de soupape est une servocommande.

13. Dispositif selon la revendication 9, dans lequel le dispositif de commande est un dispositif de commande analogique.

14. Dispositif selon la revendication 9, dans lequel ledit actionneur comporte un engrenage à vis sans fin accouplé mécaniquement à un engrenage droit.

15. Dispositif selon la revendication 1, dans lequel ledit logement intérieur comporte au moins deux orifices pour permettre l'écoulement de fluide entre des régions de côtés opposés dudit cylindre intérieur.

16. Dispositif selon la revendication 1, dans lequel ledit logement intérieur comporte au moins deux rainures dans lesquelles le fluide peut s'écouler.

17. Dispositif selon la revendication 1, dans lequel ledit logement extérieur est cylindrique.

18. Dispositif selon la revendication 17, dans lequel ledit logement intérieur est cylindrique.

19. Dispositif selon la revendication 18, comportant par ailleurs :
(a) un capteur de température positionné pour capter la température d'un fluide s'écoulant au travers de la section de sortie dudit canal d'écoulement et pour transmettre un signal de température indiquant la température d'un fluide s'écoulant au travers de la section de sortie dudit canal d'écoulement ;
(b) un dispositif de commande connecté fonctionnellement pour recevoir ledit signal de température en provenance dudit capteur et pour transmettre un signal de commande en réponse audit signal de température ; et
(c) un actionneur de soupape connecté fonctionnellement à ladite soupape et audit dispositif de commande pour commander le degré de fermeture de ladite soupape en réponse audit signal de commande.
